# EUROPÄISCHE PATENTSCHRIFT

(11) **EP 1 474 378 B1**
(45) Veröffentlichungstag und Bekanntmachung des Hinweises auf die Patenterteilung: **07.09.2005**
(21) Anmeldenummer: 03714720.4
(22) Anmeldetag: 30.01.2003
(51) Int. Cl.: C07C 209/36, C07C 209/86

(54) **VERFAHREN ZUR HERSTELLUNG VON AMINEN**
METHOD FOR THE PRODUCTION OF AMINES
PROCEDE DE PRODUCTION D'AMINES

(30) Priorität: 06.02.2002 DE 10204700
(43) Veröffentlichungstag der Anmeldung: 10.11.2004
(73) Patentinhaber: BASF Aktiengesellschaft, 67056 Ludwigshafen (DE)
(72) Erfinder: VANOPPEN, Dominic, B-2950 Kapellen (BE); SCHWAB, Ekkehard, 67434 Neustadt (DE); VAN LAAR, Frederik, 67117 Limburgerhof (DE); VOSS, Hartwig, 67227 Frankenthal (DE); OEHLENSCHLÄGER, Steffen, 67063 Ludwigschafen (DE); MACKENROTH, Wolfgang, 67098 Bad Dürkheim (DE); MORGENSCHWEIS, Konrad, 01108 Dresden (DE); PENZEL, Ulrich, 01945 Tettau (DE); WEIDNER, Bernd, 01994 Wormlage (DE)
(86) Internationale Anmeldenummer: PCT/EP2003/000924
(87) Internationale Veröffentlichungsnummer: WO 2003/066571

(56) Entgegenhaltungen:
- EP-A- 0 052 719
- WO-A-00/35852
- DE-A- 2 835 943
- DATABASE WPI Section Ch, Week 200318 Derwent Publications Ltd., London, GB; Class E14, AN 2003-176140 XP002241467 & CN 1 377 875 A (UNIV NANJING POLYTECHNIC), 6. November 2002 (2002-11-06)

## Beschreibung

Die Erfindung betrifft ein kontinuierliches Verfahren zur Herstellung von Aminen, insbesondere aromatischen Aminen, durch katalytische Hydrierung der den Aminen zugrunde liegenden Nitroverbindungen.

Die Herstellung von Aminen, insbesondere von aromatischen Mono- und/oder Polyaminen durch katalytische Hydrierung der entsprechenden Mono- und/oder Polynitroverbindungen ist seit langem bekannt und vielfach in der Literatur beschrieben.

Bei der in der Technik üblichen Herstellung der aromatischen Mono- und/oder Polyamine durch Umsetzung von Nitroverbindungen mit Wasserstoff wird eine beträchtliche Wärmemenge frei. Zumeist wird die Hydrierung daher in der Technik bei möglichst niedrigen Temperaturen unter Einsatz von Hydrierkatalysatoren in der Flüssigphase durchgeführt. Dabei wird die zu reduzierende Verbindung in einem Lösungsmittel mit dem Katalysator vermischt und diskontinuierlich in einem Autoklaven oder kontinuierlich in einem Schlaufenreaktor, einer Blasensäule oder einer Reaktorkaskade reduziert. Bei diesen bisher bekannten Verfahren gibt es eine Reihe von Nachteilen, wie z.B. die Notwendigkeit des Austragens und besonders des Ausschleusens desaktivierter Katalysatoranteile, was zu Katalysatorverlusten führt. Ferner stellen die häufig auftretenden Nebenreaktionen, die zur Bildung störender Substanzen, wie z.B. teerartiger Bestandteile, und damit zu Ausbeuteminderungen führen, ein Problem vieler bislang verwendeter Verfahren dar.

In EP-A-634 391 wird ein Verfahren zur Hydrierung von aromatischen Polynitroverbindungen zu Aminen beschrieben, in dem durch technologische Optimierung unter Einsatz eines Loop-Venturi-Reaktors mit einem Ejektor, gekoppelt mit speziellen Bedingungen wie genauem Umwälzvolumenverhältnis, genauem Energieeintrag, einem genau eingestellten Wasserstoffvolumenstrom, die genannten Probleme der Hydrierung von aromatischen Polynitroverbindungen minimiert werden sollen. Als Katalysatoren werden bekannte Hydrierkatalysatoren verwendet, wobei vorzugsweise Metalle der VIII. Nebengruppe des Periodensystems und insbesondere Raney-Eisen, -Kobalt und -Nickel eingesetzt werden.

Bei diesem Verfahren kann es, bedingt durch die Anordnung eines Wärmetauschers zur Abführung der Reaktionswärme außerhalb des Schlaufenreaktors, im Ejektor und im Reaktor zu örtlichen Überhitzungen mit sofortigem Einsetzen von Nebenreaktionen wie Kernhydrierungen, hydrogenolytischen Spaltungen bzw. Bildung von hochmolekularen, teerartigen Produkten, die die Katalysatoroberfläche belegen, kommen. Darüber hinaus stellt sich im Reaktorvolumen außerhalb des Ejektors eine bezüglich des Strömungs- und Verweilzeitverhaltens reine Blasensäulencharakteristik ein, in dem regellose klein- und großräumige Wirbel mit vergleichsweise geringer Stoffübergangsleistung auftreten. Eine wesentliche Verbesserung der Hydrierausbeute, der Hydrierselektivität und der Raum-Zeit-Ausbeute wird somit bei diesem Verfahren kaum erreicht. Außerdem wird auch hier durch das Umpumpen der gesamten Reaktionsmischung der Katalysator mechanisch stark beansprucht, was wiederum zu einer verminderten Standzeit des Katalysators führt.

In WO 00/35852 wird ein Verfahren zur Herstellung von Aminen durch Hydrierung von Nitroverbindungen beschrieben. Bei diesem Verfahren wird die Reaktion in einem vertikalen Reaktor mit einer nach unten gerichteten Strahldüse, über die die Edukte sowie das Reaktionsgemisch zugeführt werden, einen äußeren Kreislauf, über den das Reaktionsgemisch der Strahldüse zugeführt wird, sowie einer Strömungsumkehr am unteren Ende des Reaktors durchgeführt. Der Austrag des Endprodukts erfolgt vorzugsweise über eine Abtrenneinheit für den Katalysator. Als Abtrenneinheit werden beispielsweise Settler, Filtereinheiten, oder Zentrifugen vorgeschlagen.

Mit diesem Verfahren kann die Selektivität und die Raum-Zeit-Ausbeute bei Hydrierungen deutlich gesteigert werden. Für die großtechnische Hydrierung ist jedoch eine weitere Verbesserung des Verfahrens wünschenswert. Insbesondere die möglichst vollständige Abtrennung der eingesetzten Hydrierkatalysatoren ist für die Wirtschaftlichkeit des Verfahrens von entscheidender Bedeutung. Eine vollständige Abtrennung des Katalysators von der aus dem Reaktor ausgeschleusten Reaktionsmischung vereinfacht die Aufarbeitung des Endprodukts. Der abgetrennte Katalysator kann dem Reaktor wieder zugeführt werden und braucht somit nicht durch frischen Katalysator ersetzt werden. Dies ist insbesondere bei der Verwendung von Edelmetallkatalysatoren von Bedeutung.

Es ist bekannt, Katalysatoren mittels einer Querstromfiltration abzutrennen. Diese Art der Abtrennung führt zu einer besonders schonenden Abtrennung des Katalysators.

In DE 32 45 318 wird die Abtrennung von Katalysatoren bei Gas/Flüssigreaktionen mittels eines nach dem Querstromprinzip betriebenen Mikrofilters durchgeführt. Um die Beanspruchung des Katalysators gering zu halten, wird die Filtration bei Betriebsdrücken von mindestens 10 bar auf der Suspensionsseite und Differenzdrücken zwischen Suspensions- und Filtratseite von höchstens 6 bar sowie Temperaturen im Bereich zwischen 80 und 200°C betrieben.

In DE 30 40 631 wird die Entfernung von Katalysatoren aus Reaktionsmischungen mittels Membranfiltration beschrieben und erwähnt, dass dieses Verfahren auch bei der Hydrierung von Nitroaromaten eingesetzt werden kann. Als Filter werden Hohlfasern eingesetzt. Die Filtration wird bei sehr niedrigen Temperaturen durchgeführt.

Aufgabe der Erfindung war es, ein Verfahren zur Abtrennung von Katalysatoren bei der Hydrierung von Nitroaromaten zu aromatischen Aminen zu entwickeln, das eine vollständige und schonende Abtrennung der Katalysatoren erlaubt und bei dem der abgetrennte Katalysator vollständig aus der Trennstufe wieder in den Reaktor zurückgeführt werden kann.

Die Aufgabe konnte überraschenderweise gelöst werden, indem die Abtrennung des Katalysators mittels eines Querstromfilters vorgenommen wird, der als Membranfilter ausgestaltet ist, wobei die Membranfiltration bei einem Druck auf der Suspensionsseite von 5 bis 50 bar, vorzugsweise 10 bis 30 bar, einer Druckdifferenz zwischen der Suspensionsseite und der Permeatseite von mindestens 0,3 bar und einer Strömungsgeschwindigkeit auf der Suspensionsseite von 1 bis 6 m/s durchgeführt wird.

Gegenstand der Erfindung ist somit ein Verfahren zur Herstellung von Aminen durch katalytische Hydrierung von Nitroaromaten und nachfolgende Abtrennung der Katalysatoren aus dem Reaktionsgemisch, enthaltend mindestens ein aromatisches Amin und Wasser, dadurch gekennzeichnet, dass die Abtrennung der Katalysatoren kontinuierlich mittels Membranfiltration erfolgt, wobei die Membranfiltration bei einem Druck auf der Suspensionsseite von 5 bis 50 bar, einer Druckdifferenz zwischen der Suspensionsseite und der Permeatseite von mindestens 0,3 bar und einer Strömungsgeschwindigkeit auf der Suspensionsseite von 1 bis 6 m/s durchgeführt wird.

Unter Suspensionsseite wird die Seite des Membranfilters verstanden, auf der sich die Katalysator enthaltende Mischung befindet, unter Permeatseite wird die Seite des Membranfilters verstanden, auf der sich die katalysatorfreie Mischung befindet.

Zur Durchführung des erfindungsgemäßen Verfahrens und Gewinnung eines katalysatorfreien Produktstromes wird der Austrag aus dem Hydrierreaktor unter Druck mit einer Membran in Kontakt gebracht und Permeat (Filtrat) auf der Rückseite der Membran bei einem geringeren Druck als auf der Seite, auf der sich das katalysatorhaltige Reaktionsgemisch befindet, abgezogen. Man erhält ein Katalysatorkonzentrat (Retentat), das ohne weitere Aufarbeitung in den Synthesereaktor zurückgeführt werden kann, und ein praktisch katalysatorfreies Permeat, welches das Umsetzungsprodukt, beim erfindungsgemäßen Verfahren das aromatische Amin, sowie Wasser und gegebenenfalls Lösungsmittel enthält.

Bei der kontinuierlichen Durchführung des Verfahrens wird ständig zumindest ein Teilstrom des Reaktionsgemisches durch einen Membranfilter gefahren. Bei dieser Ausführungsform des erfindungsgemäßen Verfahrens ist es bevorzugt, den Membranfilter in den externen Kreislauf eines Umlaufreaktors anzuordnen. Diese Ausführungsform des erfindungsgemäßen Verfahrens ist bevorzugt.

Die für das erfindungsgemäße Verfahren eingesetzten Filtermembranen haben, abhängig von der Partikelgröße des eingesetzten Katalysators, vorzugsweise Porendurchmesser im Bereich zwischen 10 nm und 20 µm, insbesondere im Bereich zwischen 50 nm und 10 µm und vorzugsweise zwischen 100 nm und 5 µm.

Die Trennschichten der Filtermembranen können aus organischen Polymeren, Keramik, Metall, Kohlenstoff oder Kombinationen daraus bestehen und müssen in dem Reaktionsmedium und bei der Prozesstemperatur stabil sein. Aus mechanischen Gründen sind die Trennschichten in der Regel auf einer ein- oder mehrschichtigen porösen Unterstruktur, die aus dem gleichen oder auch aus mindestens einem unterschiedlichen Material wie die Trennschicht besteht, aufgebracht. Bevorzugt sind wegen der hohen Synthesetemperatur und der damit hohen Temperatur der filtrierten Reaktionsmischung anorganische Membranen. Beispiele sind Trennschichten aus Metall und Unterstrukturen aus Metall, Trennschichten aus Keramik und Unterstrukturen aus Metall, Keramik oder Kohlenstoff, Trennschichten aus Polymeren und Unterstrukturen aus Polymer, Metall, Keramik oder Keramik auf Metall. Als Keramik werden beispielsweise α-Al₂O₃, γ-Al₂O₃, ZrO₂, TiO₂, SiC oder gemischte keramische Werkstoffe eingesetzt. Als Polymere werden beispielsweise Polytetrafluorethylen, Polyvinylidenfluorid (PVDF), Polysulfon, Polyethersulfon, Polyetheretherketon, Polyamid eingesetzt.

Die Membranen werden üblicherweise in druckfeste Gehäuse eingesetzt, welche die Trennung zwischen Retentat (katalysatorhaltig) und Permeat (katalysatorfreies Filtrat) bei den für die Filtration erforderlichen Druckbedingungen erlauben. Die Gehäuse können in Flach-, Rohr-, Multikanalelement-, Kapillar- oder Wickelgeometrie ausgeführt werden, für die entsprechende Druckgehäuse, die eine Trennung zwischen Retentat und dem Permeat erlauben, verfügbar sind. Je nach Flächenbedarf kann ein Filterelement mehrere Kanäle enthalten. Weiterhin können mehrere dieser Elemente in einem Gehäuse zu einem Modul zusammengefasst werden.

In einer bevorzugten Ausführungsform werden Metallmembranen verwendet, die mit den Gehäusen verschweißt sind.

Es ist bevorzugt, das Verfahren so zu betreiben, dass sich möglichst keine Deckschichten auf der Suspensionsseite der Membran ausbilden. Falls sich störende Deckschichten ausbilden, durch die die Filtration beeinträchtigt wird, ist es möglich, diese durch Strömungsumkehr zwischen Suspensionsseite und Permeatseite zu entfernen. Die Strömungsumkehr kann insbesondere durch Anheben des Perzneatdrucks über den Retentatdruck bewirkt werden.

Die optimalen transmembranen Drücke zwischen Retentat und Permeat liegen im wesentlichen, abhängig von Durchmesser der Membranporen, den hydro-dynamischen Bedingungen, die den Deckschichtaufbau beeinflussen, und der mechanischen Stabilität der Membran bei der Betriebstemperatur je nach Membranart bei mindestens 0,3 bar, insbesondere zwischen 0,5 und 50 bar vorzugsweise 1 bis 25 bar.

Höhere transmembrane Drücke führen zumeist zu höheren Permeatflüssen. Da der Syntheseaustrag zumeist direkt der Membranfiltrationsstufe mit den Synthesedruck zugeführt wird, kann der transmembrane Druck durch Anhebung des Permeatdruckes auf einen Wert abgesenkt werden, der kleiner als der Synthesedruck ist.

Da die Synthesetemperatur durch das Verfahren vorgegeben ist und über 80°C liegt, muss die Membran bei dieser Temperatur stabil sein. Höhere Temperaturen führen prinzipiell zu höheren Permeatflüssen und sind daher bevorzugt.

Falls bei speziellen Einsatzfällen des erfindungsgemäßen Verfahrens Membranen eingesetzt werden müssen, die bei diesen Temperaturen nicht stabil sind, muss die Reaktionsmischung vor der Filtration abgekühlt und das Retentat vor der Zuführung in den Reaktor wieder erhitzt werden. Diese Ausführungsform ist nicht bevorzugt.

Die erreichbaren Permeatflüsse sind stark von der eingesetzten Membranart und Membrangeometrie, von den Prozessbedingungen, von der Suspensionszusammensetzung, von der Katalysatorkonzentration und vom Katalysatortyp abhängig. Die Flüsse liegen üblicherweise zwischen 20 und 500 kg/m2/h.

Durch das erfindungsgemäße Verfahren kann eine Katalysatorrückhaltung > 99 % erzielt werden.

Nach dem erfindungsgemäßen Verfahren lassen sich alle für die Hydrierung von Nitroaromaten einsetzbaren Katalysatoren abtrennen. Geeignete Katalysatoren sind Metalle der VIII. Nebengruppe des Periodensystems, die auf Trägermaterialien wie Kohlenstoff oder Oxiden des Aluminiums, des Siliciums oder anderer Materialien aufgebracht sein können. Vorzugsweise werden Raney-Nickel und/oder geträgerte Katalysatoren auf Basis von Nickel, Palladium, Iridium und/oder Platin auf Kohlenstoffträgern verwendet. Besonders vorteilhaft lässt sich das Verfahren zur Abtrennung von Katalysatoren einsetzen, an denen wenig der als Nebenprodukt bei der Hydrierung anfallenden hochkondensierten Nebenprodukte, häufig als "Teer" bezeichnet, anfallen. Dieser Teer kann zu Verblockungen der eingesetzten Membran führen und die Lebensdauer des Filters herabsetzen. Da Edelmetallkatalysatoren besonders selektiv arbeiten und bei der Hydrierung von Nitroaromaten nur sehr wenig höhermolekularen "Teer" erzeugen, ist die Abtrennung der Edelmetallkatalysatoren mittels Membranfiltration besonders vorteilhaft.

Die mittlere Korngröße der eingesetzten Katalysatoren liegt zumeist im Bereich zwischen 10 nm und 200 µm, insbesondere im Bereich zwischen 50 nm und 100 µm und vorzugsweise zwischen 100 nm und 30 µm.

Die Hydrierung der aromatischen Nitroverbindungen kann nach üblichen und bekannten Verfahren erfolgen.

Dabei wird im Reaktor vorzugsweise, unabhängig von der Art der eingesetzten Nitroverbindungen, ein Druck von 5 bis 50 bar, bevorzugt 10 bis 30 bar, und eine Betriebstemperatur von 80 bis 200°C, bevorzugt 100 bis 180°C, aufrechterhalten.

Die Mono- und/oder Polynitroverbindung kann hierbei in reiner Form, als Mischung mit dem entsprechenden Mono- und/oder Polyamin, als Mischung mit dem entsprechenden Mono- und/oder Polyamin und Wasser oder als Mischung mit dem entsprechenden Mono- und/oder Polyamin, Wasser und einem insbesondere alkoholischen Lösungsmittel eingesetzt. Die aromatische Mono- und/oder Polynitroverbindung wird fein verteilt in das Gemisch eingetragen. Das Reaktionsgemisch, welches den Reaktor verlässt, enthält Wasser, welches als Nebenprodukt bei der Hydrierung entsteht.

Als Reaktoren werden die für die üblichen und bekannten Hydrierreaktoren verwendet. Beispiele hierfür sind Rührkessel, Blasensäulen, die Packungen enthalten können, oder Schlaufenreaktoren, wie Loop-Venturi-Reaktoren, oder Strahlschlaufenreaktoren mit innerem und äußerem Kreislauf, wie beispielsweise in WO 00/35852 beschrieben.

Besonders vorteilhaft kann das erfindungsgemäße Verfahren zur Abtrennung von Katalysatoren bei der Verwendung von Schlaufenreaktoren mit einem äußeren Kreislauf eingesetzt werden. Hierbei wird der Membranfilter im äußeren Kreislauf angeordnet.

In einer besonders bevorzugten Ausführungsform des erfindungsgemäßen Verfahrens wird ein Hydrierreaktor eingesetzt, wie er in WO 00/35852 beschrieben ist. Bei dieser Ausführungsform kann auf eine zusätzliche Pumpe für die Membranfiltration verzichtet werden, da durch die Pumpe für den äußeren Kreislauf der notwendige Druck auf der Suspensionsseite gehalten werden kann. Dadurch kann das Verfahren deutlich vereinfacht werden. Außerdem ist bei der Verwendung derartiger Reaktoren ein vollständiger Umsatz der Nitroaromaten möglich, so dass sich die nachfolgende Aufarbeitung nach der vollständigen Abtrennung des Katalysators besonders einfach gestaltet. Bei diesem Verfahren kommen die Vorteile der Edelmetallkataysatoren, insbesondere solcher auf der Basis von Platin, Palladium und/oder Iridium, nämlich die hohe Aktivität und die gute Selektivität, besonders zum Tragen.

Vorzugsweise werden im Rahmen des erfindungsgemäßen Verfahrens aromatische Nitroverbindungen mit einer oder mehreren Nitrogruppen und 6 bis 18 C-Atomen, beispielsweise Nitrobenzole, wie o-, m-, p-Nitrobenzol, 1,3-Dinitrobenzol, Nitrotoluole, wie z.B. 2,4-, 2,6-Dinitrotoluol, 2,4,6-Trinitrotoluol, Nitroxylole, wie z.B. 1,2-Dimethyl-3-, 1,2 Dimethyl-4-, 1,4-Dimethyl-2-, 1,3-Dimethyl-2-, 2,4-Dimethyl-1- und 1,3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. Tris(hydroxymethyl)nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Methylnitrotoluol, und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 4 Prozent an vicinalem DNT und 0,5 bis 1,5 % an 2,5- und 3,5-Dinitrotoluol aufweisen, zu den entsprechenden Aminen hydriert.

Die Erfindung soll an dem nachfolgenden Beispiel näher erläutert werden.

### Beispiel 1 und Vergleichsbeispiel 2

### Hydrierung von Dinitrotoluol

In einen 5-l-Strahlschlaufenreaktor mit Umwälzpumpe, Düse, Einsteckrohr und Wärmetauscher wurde Dinitrotoluol an einem geträgerten Nickelkatalysator mit einer mittleren Teilchengröße von 5 bis 10 µm bei 25 bar und 120°C hydriert. Die Aktivität des Katalysators wurde ständig anhand von Gaschromatographie-Proben verfolgt und wenn nötig, Katalysator nachdosiert. Die Konzentration des Katalysators betrug 3 Gew.-%, bezogen auf die Reaktionsmischung.

Die Abtrennung des Katalysators aus der Reaktionsmischung konnte dabei wahlweise mit einem Membranfilter (erfindungsgemäß) oder einem Settler (Vergleich) erfolgen.

### Beispiel 1 - Membranfiltration

Ein aus hochporöser Keramik bestehender Zylinder mit einer Länge von 750 mm enthielt einen in Längsrichtung verlaufenden Kanal mit einem Durchmesser von 6 mm, auf dessen Oberfläche die eigentliche filterwirksame Membran aus Zirkoniumdioxid mit einer Porengröße von 50 nm aufgebracht war. Die zu behandelnde Suspension strömte mit einer Strömungsgeschwindigkeit von 4 m/s in dem Kanal entlang der Membran, wobei ein Teilstrom die Membran als Permeat passierte und durch das Keramik-Trägermaterial abgeführt wurde. Der Transmembrandruck betrug 2 bar, der Permeatfluss betrug 440 l/m²/h.

### Vergleichsbeispiel 2 - Schwerkraftabscheider (Settler)

Ein Teilstrom des Umwälzstromes (Reaktor-Umwälzpumpe-Düse) wurde mit dem Vordruck der Umwälzpumpe in den unteren Teil eines Settlers abgezweigt und von dort mengengeregelt ohne zusätzliches Förderorgan zurück in den Reaktor geschleust.

Der eigentliche Reaktoraustrag (Settleraustrag) durchströmte aufwärts ein um 55° geneigtes Abscheiderrohr und wurde, gesteuert vom Flüssigkeitsstand beziehungsweise Gasgehalt im Reaktor, über ein Entspannungsventil ausgetragen. Die Strömungsverhältnisse in der Abscheiderlamelle waren so eingestellt, dass alle Partikel, die größer als 1 µm waren, abgeschieden wurden und in den Settlersumpf absanken, wo sie mit dem Kreislaufstrom (Settlerrückführung) wieder in den Reaktor transportiert wurden. Kleinere Teilchen wurden mit dem Endprodukt ausgeschleust.

### Ergebnisse

In zwei Versuchsreihen wurde der Reaktor einmal mit Schwerkraftabscheider (Vergleichsversuch) und einmal mit Membranfiltration (erfindungsgemäß) betrieben.

Mit Schwerkraftabscheider konnte über eine Periode von 4 Wochen eine Raum-Zeit-Ausbeute von 250-350 kg TDA/(m³·h) erreicht werden. Es wurden 400-600g Katalysator pro Tonne TDA verbraucht.

Mit Membranfiltration wurde über eine Periode von 3 Monaten eine Raum-Zeit-Ausbeute von 400 bis 500 kg TDA/(m³·h) erreicht. Es wurden 350 bis 450 g Katalysator pro Tonne TDA verbraucht.

### Beispiele 3 und 4

Es wurden die in Beispiel 1 beschriebene Vorrichtungen zur Hydrierung und zur Katalysatorabtrennung eingesetzt und die Wirksamkeit unterschiedlicher Katalysatoren bei unterschiedlicher Reaktionstemperatur bei der Hydrierung von Nitrobenzol geprüft.

### Beispiel 3

Es wurde der Katalysator aus Beispiel 1 in einer Konzentration von 2 Gew.-%, bezogen auf die Reaktionsmischung, eingesetzt.
Der Transmembrandruck betrug 1 bar, der Permeatfluss betrug 200 l/m²/h.

Bei 140°C konnte eine Raum-Zeit-Ausbeute von 800 kg/(m³·h) erreicht werden mit einer Selektivität 99,7 %, bei 180°C konnte eine Raum-Zeit-Ausbeute von 1800 kg/(m³·h) mit einer Selektivität 98,3 % erreicht werden.

### Beispiel 4

Es wurde ein Katalysator aus 5 Gew.-% Platin und 2 Gew.-% Eisen auf Aktivkohle mit einer mittleren Teilchengröße von 20 bis 30 µm in einer Konzentration von 2 Gew.-%, bezogen auf die Reaktionsmischung, eingesetzt. Der Transmembrandruck betrug 1 bar, der Permeatfluss betrug 200 l/m²/h.

Bei 140°C konnte eine Raum-Zeit-Ausbeute von 1500 kg/(m³·h) mit Selektivität 99,82 % erreicht werden. Bei 180°C konnte eine Raum-Zeit-Ausbeute von 2200 kg/(m³.h) mit einer Selektivität 99,6 % erreicht werden.

## Patentansprüche

1. Verfahren zur Herstellung von Aminen durch katalytische Hydrierung von Nitroaromaten und nachfolgende Abtrennung der Katalysatoren aus dem Reaktionsgemisch, enthaltend mindestens ein aromatisches Amin und Wasser, **dadurch gekennzeichnet, dass** die Abtrennung der Katalysatoren kontinuierlich mittels Membranfiltration erfolgt, wobei die Membranfiltration bei einem Druck auf der Suspensionsseite von 5 bis 50 bar, einer Druckdifferenz zwischen der Suspensionsseite und der Permeatseite von mindestens 0,3 bar und einer Strömungsgeschwindigkeit auf der Suspensionsseite von 1 bis 6 m/s durchgeführt wird.

2. Verfahren nach Anspruch 1 **dadurch gekennzeichnet, dass** der Druck auf der Suspensionsseite 10 bis 30 bar beträgt.

3. Verfahren nach Anspruch 1 **dadurch gekennzeichnet, dass** die Filtermembran einen Porendurchmesser im Bereich zwischen 10 nm und 20 µm aufweist.

4. Verfahren nach Anspruch 1 **dadurch gekennzeichnet, dass** die Hydrierung in einem Strahlschlaufenreaktor durchgeführt wird.

5. Verfahren nach Anspruch 1 **dadurch gekennzeichnet, dass** die Hydrierung in einem Strahlschlaufenreaktor mit äußerem und innerem Kreislauf durchgeführt wird.

6. Verfahren nach Anspruch 1 **dadurch gekennzeichnet, dass** als Katalysatoren solche eingesetzt werden, die Metalle der VIII. Nebengruppe des Periodensystems auf Trägern enthalten.

7. Verfahren nach Anspruch 1 **dadurch gekennzeichnet, dass** als Katalysatoren Platin, Palladium und/oder Iridium enthaltende Katalysatoren auf Kohlenstoffträgern eingesetzt werden.

8. Verfahren nach Anspruch 1 **dadurch gekennzeichnet, dass** die Hydrierung in einem Strahlschlaufenreaktor mit äußerem und innerem Kreislauf durchgeführt wird, als Katalysatoren Platin, Palladium und/oder Iridium enthaltende Katalysatoren auf Kohlenstoffträgern eingesetzt werden und der Membranfilter im äußeren Kreislauf des Reaktors angeordnet ist.

## Claims

1. A process for preparing amines by catalytic hydrogenation of nitroaromatics and subsequent removal of the catalysts from the reaction mixture, which contains at least one aromatic amine and water, which comprises carrying out the removal of the catalysts continuously by means of membrane filtration, which is carried out at a pressure on the suspension side of from 5 to 50 bar, a pressure difference between the suspension side and the permeate side of at least 0.3 bar and a flux rate on the suspension side of from 1 to 6 m/s.

2. A process as claimed in claim 1, wherein the pressure on the suspension side is from 10 to 30 bar.

3. A process as claimed in claim 1, wherein the filter membrane has a pore diameter in the range from 10 nm to 20 µm.

4. A process as claimed in claim 1, wherein the hydrogenation is carried out in a jet loop reactor.

5. A process as claimed in claim 1, wherein the hydrogenation is carried out in a jet loop reactor having an external and an internal circuit.

6. A process as claimed in claim 1, wherein the catalysts used comprise metals of transition group VIII of the Periodic Table on supports.

7. A process as claimed in claim 1, wherein the catalysts used comprise platinum, palladium and/or iridium catalysts on carbon supports.

8. A process as claimed in claim 1, wherein the hydrogenation is carried out in a jet loop reactor having an external and internal circuit, the catalysts used comprise platinum, palladium and/or iridium catalysts on carbon supports and the membrane filter is located in the external circuit of the reactor.

## Revendications

1. Procédé de préparation d'amines par hydrogénation catalytique de substances nitroaromatiques et séparation ultérieure des catalyseurs hors du mélange réactionnel, contenant au moins une amine aromatique et de l'eau, **caractérisé en ce que** la séparation des catalyseurs a lieu de manière continue au moyen d'une filtration à membrane, la filtration à membrane étant effectuée à une pression du côté suspension de 5 à 50 bars, à une différence de pression entre le côté suspension et le côté perméat d'au moins 0,3 bar et à une vitesse d'écoulement du côté suspension de 1 à 6 m/seconde.

2. Procédé suivant la revendication 1, **caractérisé en ce que** la pression du côté suspension est de 10 à 30 bars.

3. Procédé suivant la revendication 1, **caractérisé en ce que** la membrane du filtre présente un diamètre de pore d'une gamme comprise entre 10 nm et 20 µm.

4. Procédé suivant la revendication 1, **caractérisé en ce que** l'hydrogénation est effectuée dans un réacteur à écoulement en boucles et à jet.

5. Procédé suivant la revendication 1, **caractérisé en ce que** l'hydrogénation est effectuée dans un réacteur à écoulement en boucles et à jet comprenant un circuit extérieur et intérieur.

6. Procédé suivant la revendication 1, **caractérisé en ce que**, comme catalyseurs, on met en oeuvre ceux qui contiennent des métaux du groupe secondaire VIII du système périodique sur des supports.

7. Procédé suivant la revendication 1, **caractérisé en ce que**, comme catalyseurs, on met en oeuvre des catalyseurs contenant du platine, du palladium et/ou de l'iridium sur des supports en carbone.

8. Procédé suivant la revendication 1, **caractérisé en ce que** l'hydrogénation est effectuée dans un réacteur à écoulement en boucles et à jet comportant un circuit extérieur et intérieur, **en ce que**, comme catalyseurs, on met en oeuvre des catalyseurs contenant du platine, du palladium et/ou de l'iridium sur des supports en carbone et **en ce que** le filtre à membrane est agencé dans le circuit extérieur du réacteur.
